# EUROPEAN PATENT APPLICATION

(11) **EP 4 682 236 A1**
(43) Date of publication of application: **21.01.2026**
(21) Application number: 25189587.6
(22) Date of filing: 15.07.2025
(51) Int. Cl.: C12M 1/00, C12M 1/26

(54) **CELL DETACHMENT APPARATUS**

(30) Priority: 18.07.2024 JP 2024114690
(71) Applicant: CANON KABUSHIKI KAISHA, Tokyo 146-8501 (JP)
(72) Inventor: NAKAMOTO, Atsushi, Tokyo (JP)
(74) Representative: WESER & Kollegen Patentanwälte PartmbB

(57) **Abstract**

A cell detachment apparatus that releases cells adhering to a culture surface of a culture base, from the culture surface, by tapping the culture base, the apparatus includes a tapping member for tapping the culture base, and a tapping control means for moving the tapping member and control position of the tapping member, wherein the tapping control means stops the tapping member at a position separated from the culture base.

## Description

### Technical field

The present disclosure relates to a cell detachment apparatus.

### BACKGROUND

In the regenerative medical field, a large number of cells are required, and a particularly effective and stable supply of adherent cells that constitute the majority of biological tissues is demanded. For example, adherent cells are cultured on a culture base, such as a polystyrene dish, detached from the culture base, and target cells are acquired from the detached cells through the cell collection and cell cleaning processes. To further increase the number of cells, some of the acquired cells are moved to another culture base, which means that a so-called successive operation is performed. In this series of processes, a cell detachment method is needed to collect cells at a high detachment rate without causing damage to the cells.

Japanese Patent Application Laid-Open No. 2014-113133 describes a method for removing cells from a culture vessel by hitting a vessel holder with a collided member to apply a vibration to the culture vessel.

### SUMMARY

The inventors of the present specification have found an issue on a cell detachment apparatus discussed in Japanese Patent Laid-Open No. 2014-113133 from a viewpoint of ease of replacing a culture base.

The present disclosure in its first aspect provides a cell detachment apparatus as specified in claim 1. Optional features are specified in claim 2 to 10.

The cell detachment apparatus according to the present disclosure is configured to stop a tapping member at a position separated from a culture base, whereby replacement of the culture base 1 is easily performed.

Features of the present disclosure will become apparent from the following description of embodiments with reference to the attached drawings. The following description of embodiments are described by way of example.

### BRIEF DESCRIPTION OF THE DRAWINGS

Fig. 1 is a schematic view illustrating a cell detachment apparatus according to an embodiment of the present disclosure.
Fig. 2 is a schematic cross-sectional view illustrating a cell detachment apparatus according to a first embodiment of the present disclosure.
Fig. 3 is a flowchart illustrating stopping control of a tapping member according to the first embodiment of the present disclosure.
Fig. 4 is a flowchart illustrating stopping control of a tapping member according to a second embodiment of the present disclosure.
Fig. 5 is a schematic cross-sectional view illustrating a cam shape for a cell detachment apparatus according to a third embodiment of the present disclosure.
Fig. 6 is a flowchart illustrating stopping control of a tapping member according to a fifth embodiment of the present disclosure.
Fig. 7 is a flowchart illustrating stopping control of a tapping member according to a sixth embodiment of the present disclosure.
Fig. 8 is a flowchart illustrating stopping control of a tapping member according to a seventh embodiment of the present disclosure.
Fig. 9 is a schematic view illustrating a cell detachment apparatus according to an eighth embodiment of the present disclosure.
Fig. 10 is a flowchart illustrating stopping control of a tapping member according to a comparative example.

### DESCRIPTION OF THE EMBODIMENTS

The following describes a cell detachment apparatus according to embodiments of the present disclosure, but the present disclosure is not limited thereto.

### (Cell Detachment Apparatus)

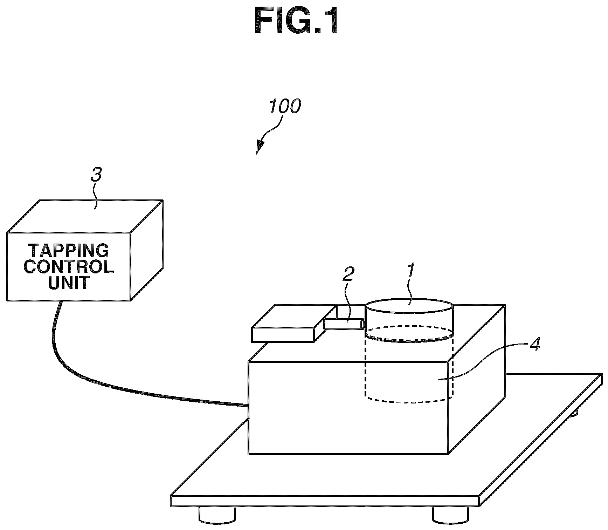

Fig. 1 is a schematic view illustrating a cell detachment apparatus 100 according to an embodiment of the present disclosure.

To detach cells adhering to the culture surface of a culture base 1 from the culture surface, the cell detachment apparatus 100 according to an embodiment described herein brings a tapping member 2 into contact with the culture base 1 to vibrate the culture base 1. The culture base 1 is placed on a disposing unit. When the tapping member 2 moves, the culture base 1 and the tapping member 2 come into contact with each other. The position and movement of the tapping member 2 are controlled by a tapping control unit 3. The movement start timing, the movement stop timing, and the driving speed of the tapping member 2 can be optionally set. When stopping the position of the tapping member 2, the tapping control unit 3 stops the tapping member 2 in a state where the tapping member 2 is separated from the culture base 1.

If the movement of the tapping member 2 is stopped in a state where the culture base 1 and the tapping member 2 are in contact with each other, the culture base 1 and the tapping member 2 interfere each other, which degrades the workability of replacement to another culture base 1 that is subjected to the next cell detachment operation. Further, the vibration of the culture base 1 after tapping may not be sufficiently transmitted. Stopping the tapping member 2 in a state where the culture base 1 and the tapping member 2 are separated from each other reduces a possibility of a leakage of a culture solution from the culture base 1 due to the culture base 1 being caught by the tapping member 2 when the culture base 1 is replaced with another one.

Since the culture base 1 and the tapping member 2 are not in contact with each other, the culture base 1 is able to be vibrated in a higher degree of freedom, which improves the cell detachment performance.

### (Tapping)

According to an embodiment described herein, the tapping member 2 taps the culture base 1 to detach at least a part of cells adhering to the culture surface of the culture base 1 from the culture surface. The tapping method that is employed in an embodiment described herein is at least one of methods including a method in which an impact force is applied to the culture base 1 by tapping the culture base 1 and another method in which the culture base 1 is moved to hit against a member of the cell detachment apparatus to tap the culture base 1. The culture base 1 may be directly tapped. If a holding vessel for holding the culture base 1 is provided, the tapping control unit 3 may apply an impact force to the culture base 1 by a method for tapping the holding vessel. According to an embodiment described herein, tapping may be performed in a periodic or aperiodic way. The intensity of the impact force to be applied to the culture base 1 can be appropriately set. According to an embodiment described herein, tapping may be continuously performed during the period from the start to the end of the cell detachment operation, completed at a timing during the period, or intermittently performed.

### (Tapping Member)

The tapping member 2 according to an embodiment described herein can be made of a material having such a weight that applies a suitable impulsive force to the culture base 1 without causing damage to the culture base 1. Examples of the shape of the tapping member 2 include a bar-like, hammer-like, and spherical shapes.

### (Tapping Control Unit)

According to an embodiment described herein, the tapping control unit 3 is not particularly limited as long as the tapping control unit 3 is able to move the tapping member 2 and control the position of the tapping member 2. For example, the tapping control unit 3 is able to control the movement of the tapping member 2 by controlling a movement unit, which moves the tapping member 2 to hit the culture base 1, to hit the culture base 1. Alternatively, the tapping control unit 3 is able to control a movement unit, which moves the culture base 1 to hit against a component of the cell detachment apparatus. Examples of movement unit include a motor and a solenoid for generating electrical power to move an object or a culture base. The tapping control unit 3 may move the tapping member 2 or the culture base 1 by using a motor and solenoid, or by using an energy accumulating member, such as a spring, together with the motor and solenoid.

The tapping control unit 3 according to an embodiment described herein may be configured to control the moving speed of the tapping member 2. The tapping control unit 3 may be configured to switch between a tapping drive mode for causing the tapping member 2 to tap the culture base 1 at a first speed, and a tapping stop mode for causing the tapping member 2 to tap the culture base 1 at a second speed lower than the first speed. With the configuration including the plurality of modes, the moving speed of the tapping member 2 is controllable by performing switching from the tapping drive mode to the tapping stop mode. For example, before stopping the movement of the tapping member 2, the tapping control unit 3 is able to lower the moving speed of the tapping member 2 by performing switching from the tapping drive mode to the tapping stop mode. After switching to the tapping stop mode, the tapping control unit 3 may move the tapping member 2 for a predetermined time period (Tk) and then stop the movement of the tapping member 2. The tapping control unit 3 is not limited to the configuration of controlling the moving speed of the tapping member 2 by mode switching but may be configured to successively change the moving speed.

The tapping control unit 3 according to an embodiment described herein is able to move the tapping member 2 by causing the tapping member 2 to perform a reciprocating movement with a constant amplitude. The reciprocating movement may be a movement reciprocating at predetermined intervals like a simple harmonic motion, or may include vibrations with a plurality of different intervals.

The tapping control unit 3 according to an embodiment described herein may be configured to control the position of the tapping member 2 to stop the tapping member 2 at a position where the distance between the tapping member 2 and the culture base 1 is at least a half of the above-described amplitude. The tapping control unit 3 may also be configured to stop the tapping member 2 at a position where the distance between the tapping member 2 and the culture base 1 is maximized in the above-described reciprocating vibration at a constant amplitude. The stop position of the tapping member 2 will be described in detail below.

### (Tapping Stopping Control)

In stopping control of the tapping according to an embodiment described herein, the tapping control unit 3 stops the tapping member 2 at a position where the tapping member 2 is separated from the culture base 1. In a case where a holding vessel for holding the culture base 1 is provided, the tapping control unit 3 stops the tapping member 2 in a state where the holding vessel and the tapping member 2 are separated from each other. A contact state between the tapping member 2 and the culture base 1 may be determined by directly observing the contact state by using a commercial camera or by detecting the positions of the tapping member 2, a part of a mechanism for applying power to the tapping member 2, and the culture base 1. In a case of moving the tapping member 2 by using a power application mechanism, such as a motor, the tapping control unit 3 controls the power application mechanism to stop the tapping member 2 at a position where the tapping member 2 does not come into contact with the culture base 1. The tapping member 2 may be stopped more than once. In a case where the tapping member 2 and the culture base 1 are in contact with each other while the tapping member 2 is stopped for the first time, the tapping control unit 3 may separate the tapping member 2 from the culture base 1 by moving again the tapping member 2 in the direction away from the culture base 1. In stopping the tapping member 2, the tapping control unit 3 may use a force different from the one in the tapping drive mode to drive the tapping member 2 and separate the tapping member 2 from the culture base 1. In this case, power may be generated by a motor that operates on electrical power, a spring that releases accumulated power, or a component that receives power in the direction away from the culture base 1 by the weight of the tapping member 2 itself.

While not particularly limited, the separation distance may be more than or equal to 1 millimeter (mm), and may be more than or equal to 3 mm. In replacement of the culture base 1, workability is improved by a distance with which the culture base 1 does not come into contact with the tapping member 2 even if the culture base 1 is shaken to a certain degree. In a case where the culture base 1 is replaced by using an automatic device, such as a robot arm, space for the arm to be inserted between the culture base 1 and the tapping member 2 can be provided.

### (Vibration Generation Unit)

The cell detachment apparatus according to an embodiment described herein may include a vibration generation unit for applying an ultrasonic vibration to the culture base 1. Examples of ultrasonic vibrations to be applied include a vibration of a frequency band between more than or equal to 10 kilohertz (kHz) and less than or equal to 1 megahertz (MHz). An ultrasonic wave generation unit is not particularly limited as long as the unit is able to apply an ultrasonic vibration to the culture base 1 and cells. For example, an ultrasonic wave oscillator, such as lead zirconate titanate (PZT) may be used as a vibrator. In a state where the vibrator is directly brought into contact with the outer surface of the culture base 1 filled with a culture medium and sealed, a vibration is able to be applied to the culture base 1. Instead of directly bringing the ultrasonic wave generation unit into contact with the culture base 1, an ultrasonic wave may be directed to detachment target cells by interposing an ultrasonic wave transmitter substance between the ultrasonic wave generation unit and a processing target region.

The vibration generation unit according to an embodiment described herein includes at least an ultrasonic vibrator and a vibrating plate. The vibration generation unit is not particularly limited as long as the unit generates an ultrasonic vibration. Examples of the vibration generation unit include a unit formed of a piezoelectric body and a vibrating plate bonded to each other. In a case where the piezoelectric body has a circular shape, the vibrating plate can be made of any one of glass, stainless use steel (SUS), and quartz. The vibrating plate made of glass, SUS, or quartz, the vibrating plate is able to output a large amplitude at a relatively high drive frequency (vibration frequency) in the ultrasonic range without being damaged as an ultrasonic vibrator.

In a case where the ultrasonic vibrator is a piezoelectric body having a ring shape, the outer diameter of the vibrating plate is equal to the outer diameter of the piezoelectric body. As for determining a thickness, the ultrasonic vibrator, in which the piezoelectric body and the vibrating plate are bonded together, having such a configuration that in bending vibration of the piezoelectric body, the midpoint in the bending thickness direction (i.e., the neutral surface subjected to neither tension nor compression during bending) exists on the vibrating plate is useful because a distortion of the piezoelectric body is able to be efficiently utilized for bending.

A commercial Langevin vibrator or a rectangular vibrator may also be used as the ultrasonic vibrator of an embodiment described herein. An example of a Langevin vibrator is the one including a piezoelectric body held by two different metal blocks which are integrally fastened by bolts, and examples of manufactures of such a Langevin vibrator include HONDA ELECTRONICS Co., LTD. and FUJI CERAMICS CORPORATION.

### (Position Acquisition Unit)

The cell detachment apparatus according to an embodiment described herein may include a position acquisition unit for acquiring information about the position of the tapping member 2. The information about the position of the tapping member 2 may be the position of the tapping member 2 itself or the position relative to the position of a component of the cell detachment apparatus (e.g., the disposing unit of the culture base 1 or the ultrasonic wave generation unit) as a reference position.

The above-described tapping control unit may be configured to control the stop position of the tapping member 2 based on the information about the position of the tapping member 2 acquired by the position acquisition unit.

### (Cells)

According to an embodiment described herein, cells are not particularly limited as long as in-vitro adherent cell culture using a culture apparatus is possible. Examples of cells include Chinese hamster ovary cells (CHO cells), mouse connective tissue L929 cells, mouse skeletal muscle myoblast cells (C2C12 cells), human embryo lung normal diploid fibroblast cells (TIG-3 cells), human embryo kidney cells (HEK293 cells), human alveolar base epithelium glandular cancer cells (A549 cells), mouse macrophage cells (RAW264.7), human cervical cancer HeLa cells, and other various cell lines; epithelial cells and endothelial cells forming biomedical tissues and organs; skeletal muscle fiber cells, smooth muscle cells, and cardiac muscle cells having contractility; neuronal cells, glia cells, and fibroblast cells configuring the nervous system; and liver parenchyma cells, liver non-parenchyma cells, and fat cells related to organic metabolism. Examples of cells having differentiation potency include induced pluripotent stem cells (iPS cells), embryo stem cells (ES cells), embryo generative cells (EG cells), embryo cancer cells (EC cells), mesenchymal stem cells, liver stem cells, pancreas stem cells, skin stem cells, muscle stem cells, generative stem cells, and other stem cells; precursor cells of each tissue; and cells differentiation-induced from these cells.

Cells according to an embodiment described herein include independent cells (what is called single cells) and a sheet-like cell culture material (cell sheet). The cell detachment method according to an embodiment described herein is useful for cells with a strong bonding force, cells having a high attachment force to the culture base 1, and cells having high trypsin sensitivity. Based on the needs of mass cell culture, for example, the cell detachment method is particularly useful for CHO cells to be used for protein production and mesenchymal stem cells applicable to cell treatment.

### (Culture Bases)

The culture base according to an embodiment described herein means the culture base 1 that is used for cell culture. The culture base 1 is not particularly limited as long as the culture base is a cell adherent culture base. Examples of culture bases include a flask, flask for tissue culture, dish, petri dish, dish for tissue culture, multi-dish, micro plate, multi-well plate, multi-plate, culture bag, and bottle.

According to an embodiment described herein, the material of the culture base 1 needs to be chemically stable and capable of culturing selected cells. Examples of the materials of the culture vessel include polyethylene, polypropylene, polycarbonate, polystyrene, polyvinyl chloride, nylon, polyurethane, polyurea, polylactic acid, polyglycolic acid, polyvinyl alcohol, polyvinyl acetate, poly(meta)acrylic acid, poly(meta)acrylic acid derivative, polyacrylonitrile, poly(meta)acrylamide, poly(meta)acrylamide derivative, polysulfone, cellulose, cellulose derivative, polysilicone, polymethylpentene, glass, and metals. In particular, polystyrene is can be used.

### (Buffered Solutions)

According to an embodiment described herein, a buffered solution can be used without limitations as long as a neutral region can be maintained. Examples of applicable buffered solutions include a Tris buffered solution, such as a Tris-HCl buffered solution, phosphate buffered solution, HEPES buffered solution, citric acid-phosphate buffered solution, glycylglycine-sodium hydroxide buffered solution, Britton-Robinson buffered solution, GTA buffered solution. In particular, a phosphate buffered solution close to the in vivo environment can be used. A phosphate buffered saline (PBS) containing a phosphate buffered solution adjusted to be isotonic with the intracellular fluid can be used.

### (Culture Media)

The culture medium type is not particularly limited. Examples of culture medium types include Dulbecco's Modified Eagles's Medium (DMEM), Ham's Nutrient Mixture F12 Medium, DMEM/F12 Medium, McCoy's 5A Medium, Eagles's Minimum Essential Medium (EMEM), alpha Modified Eagles's Minimum Essential Medium (α MEM), Minimum Essential Medium (MEM), RPMI1640 Medium, Iscove's Modified Dulbecco's Medium (IMDM), MCDB131 Medium, William Medium E, IPL41 Medium, Fischer's Medium, StemSpan H3000 (STEMCELL Technologies), StemSpanSFEM (STEMCELL Technologies), Stemlinell (Sigma-Aldrich Co. LLC), Endothelial Cell Growth Medium 2 Kit (PromoCell GmbH), Mesenchymal Stem Cell Growth Medium 2 (PromoCell GmbH), MSCGM Bullet Kit (Lonza), mTeSR1 or mTeSR2 Medium (STEMCELL Technologies), REPRO FF or REPRO FF2 (REPROCELL Inc.), NutriStem Medium (Biological Industries), and MF-Medium Mesenchymal Stem Cell Growth Medium (TOYOBO CO., LTD.).

A culture medium appropriate for each cell culture can be used from among these culture media.

### (Serums)

Serums and antibiotics may be added to the above-described culture media. Examples of serums include a fetal bovine serum (FBS), child bovine serum, adult bovine serum, equine serum, sheep serum, goat serum, swine serum, avian serum, rabbit serum, and human serum. FBS is generally frequently used because of the ease of acquisition. A serum-free medium not containing untreated or unpurified serum but containing purified blood components or animal tissue components (growth factors) is also applicable.

### (Antibiotics)

Examples of antibiotics that is added to the culture medium include penicillin, streptomycin, ampicillin, carbenicillin, tetracycline, bleomycin, actinomycin, kanamycin, actinomycin D, and amphotericin B.

### (Cell Culture Conditions)

Cell culture conditions may be appropriately selected according to the cells to be cultured. Generally, an appropriate culture medium is added in a dish, and cells with a density of 1.0 × 10¹ to 5.0 × 10⁴ cells/square centimeters (cm²) are seeded in the dish and cultured in an environment at 37 degrees Celsius (°C) with 5 percentage (%) carbon dioxide (CO₂) concentration. In this case, cells are cultured until the cell occupation area rate in the culture base 1 reaches about 70 to 80%, i.e., what is called the sub confluent state is attained.

### [Embodiments]

Embodiments of the present disclosure will be described below. Unless otherwise specifically described, the scope of the present disclosure is not limited to the embodiments.

### (CHO Cell Culture on Culture Base)

Chinese hamster ovary cells (CHO cells) were seeded in a temperature responsive vessel of 35 diameter (Φ) (UpCell 3.5 centimeters (cm) dish, CellSeed Inc.) with a density of 15,000 cells/cm² and cultured at 37°C with a 5% CO₂ concentration. As the culture medium, Ham's F12 (Thermo Fisher Scientific Inc.) with additives of 10% of Fetal Bovine Serum (Sigma-Aldrich Co. LLC) and 1% of penicillin-streptomycin (10,000 units per millimeter (U/ml), Thermo Fisher Scientific Inc.) was used. After the cells were cultured for 48 hours, the cell state was observed by using a phase-difference microscope, and a cell sheet was observed. The cell occupation area rate of the dish is about 80%.

### (A549 Cell Culture on Culture Base)

Human alveolar base epithelium glandular cancer cells (A549 cells) were seeded in a Φ35 temperature responsive vessel (UpCell 3.5 cm dish, CellSeed Inc.) with a density of 15,000 cells/cm² and cultured at 37°C with a 5% CO₂ concentration. As the culture medium, DMEM (Thermo Fisher Scientific Inc.) with additives of 10% of Fetal Bovine Serum (Sigma-Aldrich Co. LLC) and 1% of penicillin-streptomycin (10,000 U/ml, Thermo Fisher Scientific Inc.) was used. After the cells were cultured for 48 hours, the cell state was observed by using a phase-difference microscope, and a cell sheet was observed. The cell occupation area rate of the dish is about 80%.

### (C2C12 Cell Culture on Culture Base)

Mouse striated muscle cells (C2C12 cells) were seeded in a Φ35 temperature responsive vessel (UpCell 3.5 cm dish, CellSeed Inc.) with a density of 15,000 cells/cm² and cultured at 37°C with a 5% CO₂ concentration. As the culture medium, DMEM/F12 (Thermo Fisher Scientific Inc.) with additives of 10% of Fetal Bovine Serum (Sigma-Aldrich Co. LLC) and 1% of penicillin-streptomycin (10,000 U/ml, Thermo Fisher Scientific Inc.) was used. After the cells were cultured for 48 hours, the cell state was observed by using a phase-difference microscope, and a cell sheet was observed. The cell occupation area rate of the dish is about 80%.

### (MDCK Cell Culture on Culture Base)

Canine renal tubular epithelial cells (MDCK cells) were seeded in a Φ35 temperature responsive vessel (UpCell 3.5 cm dish, CellSeed Inc.) with a density of 20,000 cells/cm² and cultured at 37°C with a 5% CO₂ concentration. As the culture medium, Eagle MEM culture medium (FUJIFILM Wako Pure Chemical Corporation) with additives of 10% of Fetal Bovine Serum (Sigma-Aldrich Co. LLC) and 1% of penicillin-streptomycin (10,000 U/ml, Thermo Fisher Scientific Inc.) was used. After the cells were cultured for 48 hours, the cell state was observed by using a phase-difference microscope, and a cell sheet was observed. The cell occupation area rate of the dish is about 80%.

### (HEK293 Cell Culture on Culture Base)

Human embryo kidney cells (HEK293 cells) were seeded in a Φ35 temperature responsive vessel (UpCell 3.5 cm dish, CellSeed Inc.) with a density of 20,000 cells/cm² and cultured at 37°C with a 5% CO₂ concentration. As the culture medium, Eagle MEM culture medium (FUJIFILM Wako Pure Chemical Corporation) with additives of 10% of Fetal Bovine Serum (Sigma-Aldrich Co. LLC) and 1% of penicillin-streptomycin (10,000 U/ml, Thermo Fisher Scientific Inc.) was used. After the cells were cultured for 48 hours, the cell state was observed by using a phase-difference microscope, and a cell sheet was observed. The cell occupation area rate of the dish is about 80%.

### (BAEC Cell Culture on Culture Base)

Bovine aortic endothelial cells (BAEC cells) were seeded in a Φ35 temperature responsive vessel (UpCell 3.5 cm dish, CellSeed Inc.) with a density of 20,000 cells/cm² and cultured at 37°C with a 5% CO₂ concentration. As the culture medium, DMEM (Thermo Fisher Scientific Inc.) with additives of 10% of Fetal Bovine Serum (Sigma-Aldrich Co. LLC) and 1% of penicillin-streptomycin (10,000 U/ml, Thermo Fisher Scientific Inc.) was used. After the cells were cultured for 48 hours, the cell state was observed by using a phase-difference microscope, and a cell sheet was observed. The cell occupation area rate of the dish is about 80%.

### (hMSC Cell Culture on Culture Base)

Mesenchymal stem cells (hMSC cells) were seeded in a Φ35 temperature responsive vessel (UpCell 3.5 cm dish, CellSeed Inc.) with a density of 40,000 cells/cm² and cultured at 37°C with a 5% CO₂ concentration. As the culture medium, Mesenchymal Stem Cell Growth Medium 2 (PromoCell GmbH) with additives of 10% of Fetal Bovine Serum (Sigma-Aldrich Co. LLC) and 1% of penicillin-streptomycin (10,000 U/ml, Thermo Fisher Scientific Inc.) was used. After the cells were cultured for seven days while the culture medium was replaced at intervals of 48 hours, the cell state was observed by using a phase difference microscope, and cell attachment and growth were confirmed. The cell occupation area rate of the dish is about 80%.

### (Evaluation of Effects)

The effects were evaluated based on three different items: the workability of the replacement operation of the culture base 1, the variation in the releasing time, and the variation in the stop position of the tapping member 2. The workability of the replacement operation of the culture base 1 was evaluated based on the time duration since the time when the tapping member 2 was stopped until the time when the culture base 1 was replaced. Specific procedures include stopping the tapping member 2, taking out the placed culture base 1, and placing another culture base 1. In this processing, if the tapping member 2 and the culture base 1 came contact with each other and the culture medium in the culture base 1 overflowed, the time duration until cleaning process was performed and another culture base 1 was placed was evaluated. The cell detachment operation is performed five times for each of CHO cells, A549 cells, C2C12 cells, MDCK cells, HEK293 cells, BAEC cells, and hMSC cells. The workability of the culture base replacement was evaluated based on the longest time duration in seconds. According to an embodiment described herein, the evaluation was made based on the following criteria:
A: Very good (replacement time < 5 seconds)
B: Good (5 seconds ≤ replacement time < 10 seconds)
C: Effective (10 seconds ≤ replacement time < 15 seconds)
D: Bad (15 seconds ≤ replacement time)

The present disclosure is determined to be effective in a case where the replacement time is less than 15 seconds.

The variation in the detachment time was evaluated based on the variation in the time required for detachment. The cell detachment operation was performed five times for each of CHO cells, A549 cells, C2C12 cells, MDCK cells, HEK293 cells, BAEC cells, and hMSC cells. The standard deviation of the detachment time when each cell type was detached was divided by the average value of the five detachment operations to obtain the individual cell variation value. Then, the variation in the detachment time was evaluated based on the maximum value of the individual cell variation value. According to an embodiment described herein, the evaluation of the variation in the detachment time was evaluated based on the following criteria:
A: Very good (standard deviation/average value < 0.1)
B: Good (0.1 ≤ standard deviation/average value < 0.3)
C: Effective (0.3 ≤ standard deviation/average value < 0.5)
D: Bad (0.5 ≤ standard deviation/average value)

The present disclosure was determined to be effective in a case where the standard deviation/average value was less than 0.5.

The variation in the stop position of the tapping member 2 was evaluated based on the variation in the distance between the culture base 1 and the tapping member 2 measured when the tapping member 2 was stopped. The cell detachment operation was performed five times for each of CHO cells, A549 cells, C2C12 cells, MDCK cells, HEK293 cells, BAEC cells, and hMSC cells. The distance between the culture base 1 and the tapping member 2 measured after the five times detachment was divided by the maximum moving distance of the tapping member 2. Then, the standard deviation was calculated for the distance between the culture base 1 and the tapping member 2 divided by the maximum moving distance of the tapping member 2 obtained in stopping control performed a total of 35 times (7 cell types × 5 cell detachment operations) to evaluate the variation in the stop position. According to an embodiment described herein, the evaluation of the variation in the stop position was determined based on the following criteria:
A: Very good (0.1 < standard deviation)
B: Good (0.1 ≤ standard deviation < 0.3)
C: Effective (0.3 ≤ standard deviation < 0.5)
D: Bad (0.5 ≤ standard deviation)

The present disclosure was determined to be effective in a case where the standard deviation/average value is less than 0.5.

### (First Embodiment)

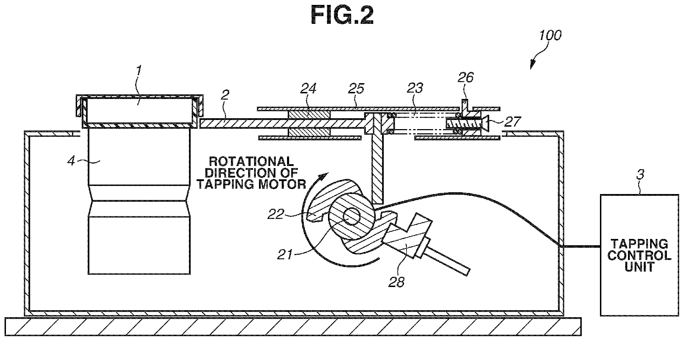

Fig. 2 is a schematic cross-sectional view illustrating a cell detachment apparatus according to a first embodiment of the present disclosure. The culture base 1 placed on an ultrasonic vibrator 4 for generating an ultrasonic vibration is tapped.

In the cell detachment operation, application of a vibration by the ultrasonic vibrator 4 illustrated in Fig. 1 is performed. Referring to Fig. 2, a tapping motor 21 connected to the tapping control unit 3 is supplied with power when tapping the culture base 1. The tapping motor 21 supplied with power starts rotating in the direction indicated by the rotational direction of the tapping motor in Fig. 2. A cam 22 connected with the tapping motor 21 is in contact with the tapping member 2 and is able to move the tapping member 2 with the rotation of the tapping motor 21. The tapping member 2 is pressed in the direction for coming into contact with the cam 22 by a tapping spring 23. The compression length of the tapping spring 23 is regulated by a compression length adjustment member 26 of which the position is adjustable by a compression length adjustment screw 27. The tapping member 2 pressed by the tapping spring 23 moves along a slider 24 to tap the culture base 1. A photo-interrupter 28 connected with the tapping control unit 3 enables detection of positional information about the cam 22, i.e., positional information about the tapping member 2. When the tapping motor 21 is applied with a 24V voltage, the tapping motor 21 turns the cam 22 at 150 revolutions per minute (rpm) to tap the culture base 1 at a frequency of 5 Hz.

The ultrasonic vibrator 4 connected with a control unit (not illustrated) is supplied with power from the control unit when an ultrasonic vibration is applied. The ultrasonic vibrator 4 is in contact with the bottom surface of the culture base 1. When supplied with power, the ultrasonic vibrator 4 vibrates the culture base 1 at an ultrasonic frequency. A bolted Langevin vibrator (HEC-30502, HONDA ELECTRONICS Co., LTD.) was used as the ultrasonic vibrator 4. A 50-kHz 20V alternating current (AC) voltage was applied to the ultrasonic vibrator 4 to vibrate the culture base 1.

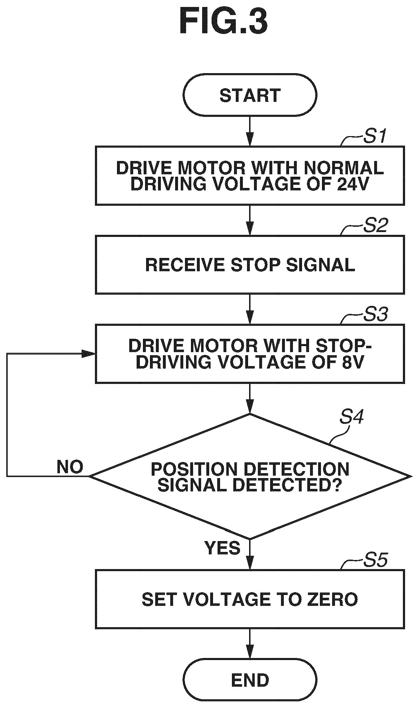

Fig. 3 is a flowchart illustrating the stopping control of the tapping member 2 according to the first embodiment of the present disclosure. The cell detachment operation was performed on CHO cells, A549 cells, C2C12 cells, MDCK cells, HEK293 cells, BAEC cells, and hMSC cells cultured on the culture base 1.

In step S1, the tapping control unit 3 applies 24V as a normal drive voltage to the tapping motor 21 to start the cell detachment operation. The voltage indicated in Fig. 3 is the voltage that is used to release CHO cells. Different voltages are used to release different cell types. More specifically, 30V was used for A549 cells, 20V was used for C2C12 cells, 30V was used for MDCK cells, 12V was used for HEK293 cells, 12V was used for BAEC cells, and 24V was used for hMSC cells. After completion of the cell detachment operation, then in step S2, the tapping control unit 3 receives a stop signal. In response to receipt of the stop signal, then in step S3, the tapping control unit 3 applies 8V as a drive stop voltage to the tapping motor 21. This changes the rotational speed of the tapping motor 21 from 150 rpm to 50 rpm. The moving speed of the tapping member 2 decreases with decrease in the motor speed. In step S4, in a state where the moving speed of the tapping member 2 is decreased by the drive stop signal, the tapping control unit 3 obtains positional information about the tapping member 2 based on the position detection signal from the photo-interrupter 28.

The detection signal is obtained when the cam 22 interrupts the light of the photo-interrupter 28, which is the moment when the tapping member 2 is separated most from the culture base 1. Immediately after the tapping control unit 3 obtains the position detection signal (YES in step S4), then in step S5, the tapping control unit 3 sets the application voltage to the tapping motor 21 to zero to stop the tapping member 2 in a state where the culture base 1 and the tapping member 2 are separated from each other.

### (Second Embodiment)

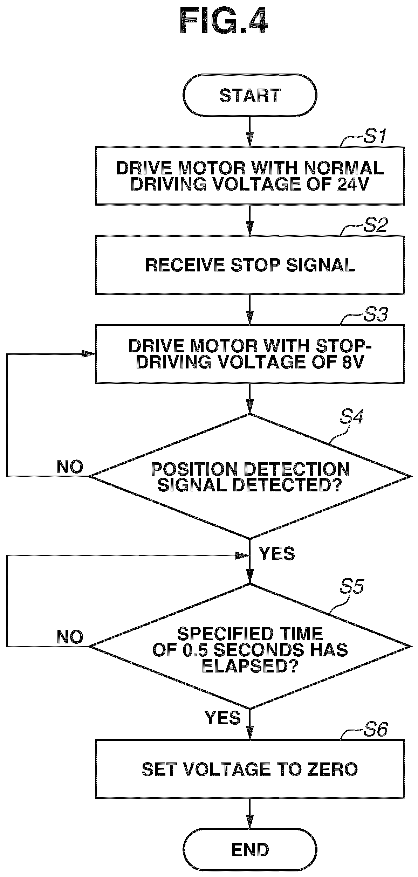

Fig. 4 is a flowchart illustrating stopping control of the tapping member 2 according to the second embodiment of the present disclosure. The cell detachment operation and the stopping control of the tapping member 2 were performed with a configuration of the cell detachment apparatus similar to the configuration of the cell detachment apparatus according to the first embodiment. In step S1, the tapping control unit 3 applies 24V as a normal drive voltage to the tapping motor 21 to start the cell detachment operation. The voltage indicated in Fig. 4 is the voltage that was used to release CHO cells like the first embodiment. Voltages for different cell types were set in a similar way to the first embodiment. After completion of the cell detachment operation, then in step S2, the tapping control unit 3 receives the stop signal. In response to receipt of the stop signal, then in step S3, the tapping control unit 3 applies 8V as a drive stop voltage to the tapping motor 21. This changes the rotational speed of the tapping motor 21 from 150 rpm to 50 rpm. The moving speed of the tapping member 2 decreases with decrease in the motor speed. In step S4, in a state where the moving speed of the tapping member 2 is decreased by the drive stop signal, the tapping control unit 3 obtains positional information about the tapping member 2 based on the position detection signal from the photo-interrupter 28. In step S5, the tapping control unit 3 drives the tapping motor 21 with the drive stop voltage until a predetermined time Tk (0.5 seconds) has elapsed. In step S6, the tapping control unit 3 sets the application voltage to the tapping motor 21 to zero to stop the tapping member 2.

In a case where the predetermined time Tk is not provided, the light of the photo-interrupter 28 is interrupted, and the photo-interrupter 28 outputs the detection signal in the stopping control from step S4 and thereon. The tapping control unit 3 receives the detection signal and determines to set the application voltage to the tapping motor 21 to zero to stop power supply to the tapping motor 21. Then, the tapping motor 21 rotates by inertia and then stops. The delay time since the time when the cam 22 interrupts the light of the photo-interrupter 28 until the time when the tapping motor 21 actually stops may vary in each stopping control. Consequently, the separation distance between the tapping member 2 and the culture base 1 may also slightly vary. Driving the tapping motor 21 for the predetermined time Tk and then stopping the tapping motor 21 are performed to eliminate the delay since the time when the cam 22 interrupts the light of the photo-interrupter 28 until the time when power supply to the tapping motor 21 is stopped, during the delay time. More specifically, the variation in the uncontrollable delay time is controlled using the predetermined time Tk, whereby more accurate control of the stop position of the tapping member 2 is performed.

### (Third Embodiment)

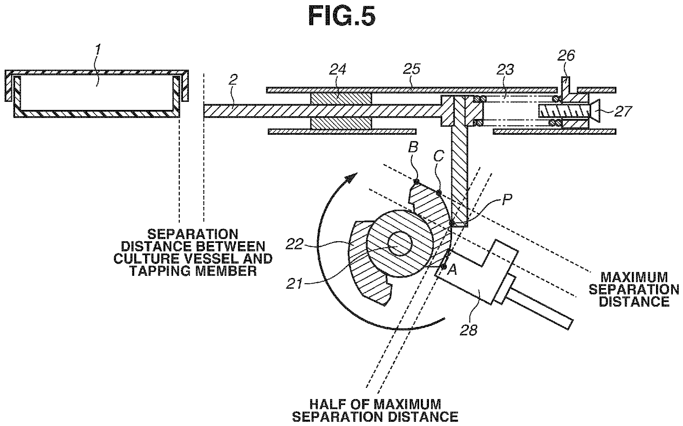

In a third embodiment of the present disclosure, the same cell detachment apparatus as the cell detachment apparatus according to the first embodiment is basically used, but the cell detachment apparatus differs from the cell detachment apparatus according to the first embodiment in the shape of the cam 22. The cell detachment operation is performed using stopping control of the tapping member 2 similar to the first embodiment. Fig. 5 illustrates the shape of the cam 22 that is used for the cell detachment apparatus according to the third embodiment of the present disclosure. When a leading edge P of the tapping member 2 is in contact with a point A of a cam 22, the separation distance between the culture base 1 and the tapping member 2 is a half of the maximum separation distance. When the leading edge P of the tapping member 2 is in contact with a point between points B and C of the cam 22, the separation distance between the culture base 1 and the tapping member 2 is the maximum separation distance. Using the cam 22 having such a shape allows the tapping member 2 and the culture base 1 to be separated by at least a half of the maximum separation distance between them as long as the leading edge P of the tapping member 2 is in contact with a point between the points B and C of the cam 22.

### (Fourth Embodiment)

In a fourth embodiment of the present disclosure, the cell detachment operation is performed using a cell detachment apparatus similar to the third embodiment.

Referring to Fig. 5, when the leading edge P of the tapping member 2 is in contact with a point between the points B and C of the cam 22, the separation distance between the tapping member 2 and the culture base 1 gradually increases as the contact point shifts from the point A to the point C. According to an embodiment described herein, the tapping member 2 is stopped in a state where the leading edge P of the tapping member 2 is in contact with a point between points A and C of the cam 22. This increases the compression length of the tapping spring 23 when the tapping member 2 is stopped in comparison with the compression length at the time of the maximum compression, whereby durability of the tapping spring 23 is improved.

### (Fifth Embodiment)

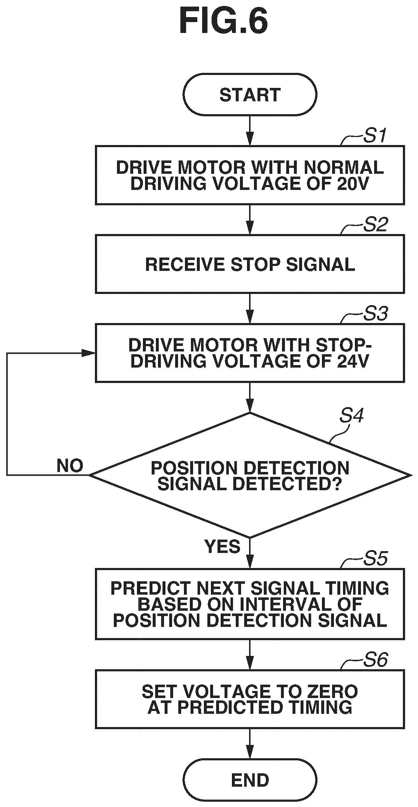

In a fifth embodiment of the present disclosure, the cell detachment operation is performed using a cell detachment apparatus similar to the cell detachment apparatus according to the first embodiment, but the cell detachment apparatus differs from the cell detachment apparatus according to the first embodiment in stopping control of the tapping member 2. Fig. 6 is a flowchart illustrating stopping control of the tapping member 2 according to an embodiment described herein. In step S1, the tapping control unit 3 applies 20V as a normal drive voltage to the tapping motor 21 to start the cell detachment operation. The voltage indicated in Fig. 6 is the voltage that is used to release CHO cells. Different voltages are used to release different cell types. More specifically, 22V was used for A549 cells, 18V was used for C2C12 cells, 22V was used for MDCK cells, 12V was used for HEK293 cells, 12V was used for BAEC cells, and 20V was used for hMSC cells. After completion of the cell detachment operation, then in step S2, the tapping control unit 3 receives the stop signal.

In response to receipt of the stop signal, then in step S3, the tapping control unit 3 applies 24V as a drive stop voltage to the tapping motor 21. This changes the rotational speed of the tapping motor 21 from 125 rpm to 150 rpm. Consequently, the moving speed of the tapping member 2 increases with increasing motor speed. In step S4, the tapping control unit 3 obtains positional information about the tapping member 2 based on the position detection signal from the photo-interrupter 28. The detection signal is obtained when the cam 22 interrupts the light of the photo-interrupter 28, which is the moment when the tapping member 2 is separated most from the culture base 1. The tapping member 2 performs a periodic motion, and the detection signal is periodically transmitted from the photo-interrupter 28. In step S5, the tapping control unit 3 predicts the timing when the next signal comes, based on the interval of the position detection signal. Since the timing when the signal comes is the timing when the culture base 1 and the tapping member 2 are separated from each other, the tapping control unit 3 estimates the stop position of the tapping member 2 due to the delay by factoring in a delay since the time when the signal is received until the time when the tapping member 2 is actually stopped. In step S6, in detachment of CHO cells according to an embodiment described herein, the tapping control unit 3 sets the application voltage to the tapping motor 21 to zero earlier than the next signal timing by 100 milliseconds (msec). Thus, the tapping member 2 is able to be stopped in a state where the tapping member 2 and the culture base 1 are separated from each other. The tapping control unit 3 performs tapping control on other cell types at an appropriate timing, whereby the tapping member 2 is stopped in a state where the tapping member 2 and the culture base 1 are separated from each other.

### (Sixth Embodiment)

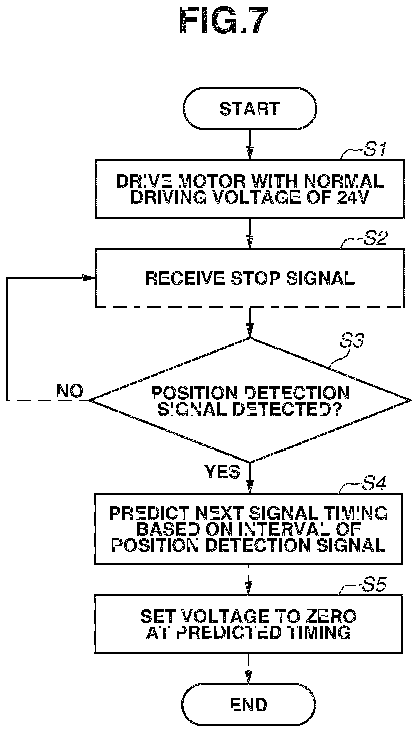

In a sixth embodiment of the present disclosure, the cell detachment operation is performed using a cell detachment apparatus similar to the cell detachment apparatus according to the first embodiment, but the cell detachment apparatus differs from cell detachment apparatus according to the first embodiment in stopping control of the tapping member 2. Fig. 7 is a flowchart illustrating stopping control of the tapping member 2 according to an embodiment described herein. In step S1, the tapping control unit 3 applies 24V as a normal drive voltage to the tapping motor 21 to start the cell detachment operation. The voltage indicated in Fig. 7 is the voltage that is used to release CHO cells like the first embodiment. Voltages for different cell types were set in a similar way to the first embodiment. After completion of the cell detachment operation, in step S2, the tapping control unit 3 receives the stop signal. In step S3, the tapping control unit 3 obtains positional information for the tapping member 2 based on the position detection signal from the photo-interrupter 28. The detection signal is obtained when the cam 22 interrupts the light of the photo-interrupter 28, and at this moment, the tapping member 2 is separated most from the culture base 1.

Since the tapping member 2 performs a periodic motion, the detection signal is periodically transmitted from the photo-interrupter 28. In step S4, the timing when the next signal comes is predicted based on the interval of the position detection signal. Since the signal comes at the timing when the culture base 1 and the tapping member 2 are separated from each other, the tapping control unit 3 estimates the stop position of the tapping member 2 due to the delay by factoring in a delay since the time when the signal is received until the time when the tapping member 2 is actually stopped. In step S5, in detachment of CHO cells according to an embodiment described herein, the tapping control unit 3 sets the application voltage to the tapping motor 21 to zero at a timing earlier than the next signal timing by 100 msec. Thus, the tapping member 2 is able to be stopped in a state where the tapping member 2 and the culture base 1 are separated from each other. The tapping control are performed on other cell types at an appropriate timing, whereby the tapping member 2 is stopped in a state where the tapping member 2 and the culture base 1 are separated from each other.

### (Seventh Embodiment)

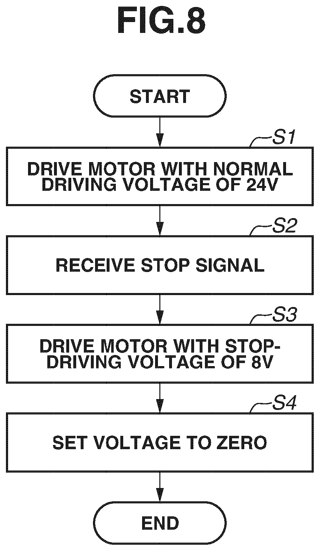

In a seventh embodiment of the present disclosure, the cell detachment operation is performed using a cell detachment apparatus similar to the cell detachment apparatus according to the first embodiment, but the cell detachment apparatus differs from the cell detachment apparatus according to the first embodiment in stopping control of the tapping member 2. Fig. 8 is a flowchart illustrating stopping control of the tapping member 2 according to an embodiment described herein. In step S1, the tapping control unit 3 applies 24V as a normal drive voltage to the tapping motor 21 to start the cell detachment operation. The voltage indicated in Fig. 8 is the voltage that is used to release CHO cells like the first embodiment. Voltages for different cell types were set in a similar way to the first embodiment. After completion of the cell detachment operation, then in step S2, the tapping control unit 3 receives the stop signal. In response to receipt of the stop signal, then in step S3, the tapping control unit 3 applies 8V as a drive stop voltage to the tapping motor 21. This changes the rotational speed of the tapping motor 21 from 150 rpm to 50 rpm. The moving speed of the tapping member 2 decreases with decrease in the motor speed. After the change in the voltage to the drive stop voltage, then in step S5, the tapping control unit 3 sets the application voltage to the tapping motor 21 to zero, whereby the tapping member 2 is stopped in a state where the culture base 1 and the tapping member 2 are separated from each other.

### (Eighth Embodiment)

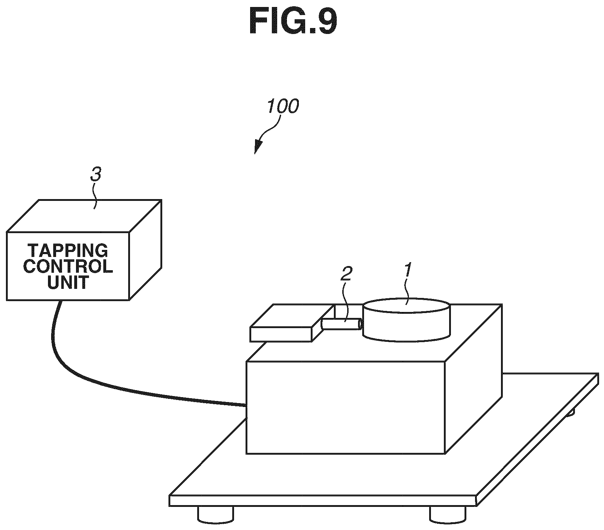

Fig. 9 is a schematic view illustrating a cell detachment apparatus according to an eighth embodiment of the present disclosure. This differs from the first embodiment in that no ultrasonic vibration is applied. The stopping control of the tapping member 2 is performed in a way similar to the stopping control according to the first embodiment.

### (Comparative Example)

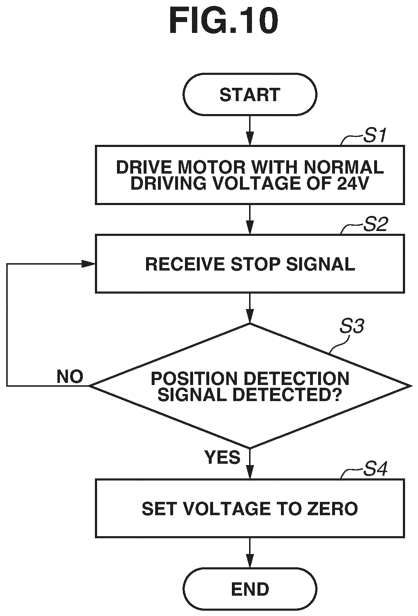

Fig. 11 is a flowchart illustrating stopping control of the tapping member 2 according to the comparative example. In step S1, the tapping control unit 3 applies 24V as a normal drive voltage to the tapping motor 21 to start the cell detachment operation. The voltage indicated in Fig. 10 is the voltage that is used to release CHO cells like the first embodiment. Voltages for different cell types were set in a similar way to the first embodiment. After completion of the cell detachment operation, then in step S2, the tapping control unit 3 receives the stop signal. In step S3, the tapping control unit 3 obtains positional information about the tapping member 2 based on the position detection signal from the photo-interrupter 28. The tapping control unit 3 obtains the detection signal when the cam 22 interrupts the light of the photo-interrupter 28, which is a timing at which the tapping member 2 is separated most from the culture base 1. Immediately after the tapping control unit 3 obtains the position detection signal in step S3, then in step S4, the tapping control unit 3 sets the application voltage to the tapping motor 21 to zero. There is a delay since the time when the detection signal is output from the photo-interrupter 28 until the time when the tapping motor 21 actually stops, and consequently, a variation occurs in the stop position depending on the communication timing and the driving speed of the tapping member 2. When a sequence of driving and stopping the cell detachment apparatus was performed five times for each cell type, the apparatus was stopped at least once in a state where the tapping member 2 and the culture base 1 were in contact with each other. Due the apparatus being stopped in the contact state, it takes at least 15 seconds to replace the culture base 1, resulting in the degraded workability of the culture base replacement. Further, when the apparatus was stopped in a state where the tapping member 2 and the culture base 1 were in contact with each other, the vibration by tapping and the vibration by the ultrasonic wave are disturbed, resulting in degradation in detachment time and degradation in the variation in the releasing time. Since the driving speed of the tapping member 2 is different for each cell type, the variation in the stop position of the tapping member 2 was also degraded.

**[Table 1]**

| | Stop position control of tapping member | Drive in predetermined time Tk | Driving speed before stopping | Tapping position detection |
|---|---|---|---|---|
| First embodiment | Stopped at position where distance from culture base is maximized | - | Lower than in drive mode | Performed |
| Second embodiment | Stopped at position where distance from culture base is maximized | Performed | Lower than in drive mode | Performed |
| Third embodiment | Stopped at position separated from culture base by at more than or equal to half of tapping member amplitude | - | Lower than in drive mode | Performed |
| Fourth embodiment | Stopped at position closer to culture base than position where distance therefrom is maximized | - | Lower than in drive mode | Performed |
| Fifth embodiment | Stopped at position where distance from culture base is maximized | - | Higher than in drive mode | Performed |
| Sixth embodiment | Stopped at position where distance from culture base is maximized | - | Same as in drive mode | Performed |
| Seventh embodiment | Stopped at position where distance from culture base is maximized | - | Lower than in drive mode | - |
| Eighth embodiment | Stopped at position where distance from culture base is maximized | - | Lower than in drive mode | Performed |
| Comparative example | None | - | Same as in drive mode | Performed |
| | Ultrasonic wave generation unit | Replacement work-ability | Variation in releasing time | Variation in stop position |
| First embodiment | Provided | A | A | B |
| Second embodiment | Provided | A | A | A |
| Third embodiment | Provided | B | A | B |
| Fourth embodiment | Provided | A | A | B |
| Fifth embodiment | Provided | B | B | C |
| Sixth embodiment | Provided | B | A | B |
| Seventh embodiment | Provided | B | A | C |
| Eighth embodiment | - | A | C | B |
| Comparative example | Provided | D | D | D |

Table 1 shows results of evaluating the ease of replacing the culture base 1 (workability of the culture base replacement), the variation in the releasing time, and the variation in the stop position of the tapping member 2 according to the first to the eighth embodiments and the comparative example of the present disclosure. According to these results, the cell detachment apparatus according to an embodiment described herein facilitates the replacement of the culture base 1 (provides high workability of the culture base replacement).

The embodiments of the present disclosure include the following methods and configurations.

### (Configuration 1)

A cell detachment apparatus that releases cells adhering to a culture surface of a culture base, from the culture surface, by tapping the culture base, the apparatus comprising a tapping member configured to tap the culture base, and a tapping control unit configured to move the tapping member and control a position of the tapping member, wherein the tapping control unit stops the tapping member at a position separated from the culture base.

### (Configuration 2)

The cell detachment apparatus according to Configuration 1, wherein the tapping control unit controls a moving speed of the tapping member.

### (Configuration 3)

The cell detachment apparatus according to Configuration 1 or 2, further comprising a vibration generation unit configured to apply an ultrasonic vibration to the culture base.

### (Configuration 4)

The cell detachment apparatus according to any one of Configurations 1 to 3, further comprising a position acquisition unit configured to acquire information about the position of the tapping member, wherein the tapping control unit controls a stop position of the tapping member based on the information about the position of the tapping member.

### (Configuration 5)

The cell detachment apparatus according to any one of Configurations 1 to 3, wherein the tapping control unit controls the moving speed of the tapping member by performing switching between a tapping drive mode in which the tapping member is caused to tap the culture base at a first speed, and a tapping stop mode in which the tapping member is caused to tap the culture base at a second speed lower than the first speed.

### (Configuration 6)

The cell detachment apparatus according to Configuration 5, wherein, before stopping the movement of the tapping member, the tapping control unit lowers the moving speed of the tapping member by performing switching from the tapping drive mode to the tapping stop mode.

### (Configuration 7)

The cell detachment apparatus according to Configuration 6, wherein, after switching to the tapping stop mode, the tapping control unit moves the tapping member for a predetermined time (Tk) and then stops the movement of the tapping member.

### (Configuration 8)

The cell detachment apparatus according to any one of Configurations 1 to 7, wherein the tapping control unit moves the tapping member in such a manner that the tapping member is reciprocally moved with a constant amplitude.

### (Configuration 9)

The cell detachment apparatus according to Configuration 8, wherein the tapping control unit controls the position of the tapping member to stop the tapping member at a position where a distance between the tapping member and the culture base is more than or equal to a half of the amplitude.

### (Configuration 10)

The cell detachment apparatus according to Configuration 8, wherein the tapping control unit stops the tapping member at a position where a distance between the tapping member and the culture base is maximized.

Various embodiments have been described in detail above but it will be understood that the present disclosure is not limited to these embodiments and encompasses all modifications, variants, alternatives and equivalents falling within the scope of the appended claims.

## Claims

1. A cell detachment apparatus that releases cells adhering to a culture surface of a culture base, from the culture surface, by tapping the culture base, the apparatus comprising:
a tapping member for tapping the culture base; and
tapping control means for moving the tapping member and control a position of the tapping member,
wherein the tapping control means stops the tapping member at a position separated from the culture base.

2. The cell detachment apparatus according to claim 1, wherein the tapping control means controls a moving speed of the tapping member.

3. The cell detachment apparatus according to claim 1, further comprising vibration generation means for applying an ultrasonic vibration to the culture base.

4. The cell detachment apparatus according to claim 1, further comprising
position acquisition means for acquiring information about the position of the tapping member,
wherein the tapping control means controls a stop position of the tapping member based on the information about the position of the tapping member.

5. The cell detachment apparatus according to claim 1, wherein the tapping control means controls the moving speed of the tapping member by performing switching between a tapping drive mode in which the tapping member is caused to tap the culture base at a first speed, and a tapping stop mode in which the tapping member is caused to tap the culture base at a second speed lower than the first speed.

6. The cell detachment apparatus according to claim 5, wherein, before stopping movement of the tapping member, the tapping control means lowers the moving speed of the tapping member by performing switching from the tapping drive mode to the tapping stop mode.

7. The cell detachment apparatus according to claim 6, wherein, after switching to the tapping stop mode, the tapping control means moves the tapping member for a predetermined time, Tk, and then stops the movement of the tapping member.

8. The cell detachment apparatus according to claim 1, wherein the tapping control means moves the tapping member in such a manner that the tapping member is reciprocally moved with a constant amplitude.

9. The cell detachment apparatus according to claim 8, wherein the tapping control means controls the position of the tapping member to stop the tapping member at a position where a distance between the tapping member and the culture base is more than or equal to a half of the constant amplitude.

10. The cell detachment apparatus according to claim 8, wherein the tapping control means stops the tapping member at a position where a distance between the tapping member and the culture base is maximized.
